# EUROPÄISCHE PATENTSCHRIFT

(11) **EP 2 473 129 B1**
(45) Veröffentlichungstag und Bekanntmachung des Hinweises auf die Patenterteilung: **20.11.2013**
(21) Anmeldenummer: 10757177.0
(22) Anmeldetag: 25.08.2010
(51) Int. Cl.: A61B 19/00, H01F 27/08

(54) **SPULENSYSTEM FÜR EINE MAGNETISCH GEFÜHRTE KAPSELENDOSKOPIE**
SOLENOID SYSTEM FOR MAGNETICALLY GUIDED CAPSULE ENDOSCOPY
SYSTÈME DE BOBINES POUR ENDOSCOPIE PAR CAPSULE À GUIDAGE MAGNÉTIQUE

(30) Priorität: 31.08.2009 DE 102009039484
(43) Veröffentlichungstag der Anmeldung: 11.07.2012
(73) Patentinhaber: Siemens Aktiengesellschaft, 80333 München (DE)
(72) Erfinder: JULOSKI, Aleksandar, 90491 Nuernberg (DE); REINSCHKE, Johannes, 90425 Nürnberg (DE)
(86) Internationale Anmeldenummer: PCT/EP2010/062365
(87) Internationale Veröffentlichungsnummer: WO 2011/023710

(56) Entgegenhaltungen:
- WO-A1-02/49705
- WO-A1-2009/089992
- US-A- 3 358 676
- US-A1- 2007 270 628

## Beschreibung

Die Erfindung betrifft ein Spulensystem, mit dessen Hilfe eine magnetisch geführte Kapselendoskopie an einem Patienten durchführbar ist.

Bei der magnetisch geführten Kapselendoskopie (MGCE) wird eine Endoskopiekapsel, welche verschiedene medizinische Aufgaben erfüllen kann, in einen Patienten eingebracht. Die Kapsel enthält ein magnetisches Element, mittels dessen durch Anlegen eines äußeren Magnetfeldes eine Kraft bzw. Drehmoment auf die Kapsel ausgeübt werden kann. Durch gezielte Krafteinwirkung kann die Kapsel im Patienten berührungslos fortbewegt werden und dort entsprechend medizinische Aufgaben erfüllen.

Das äußere Magnetfeld zur Kraftausübung auf die Kapsel wird von einem außerhalb des Patienten angeordneten sogenannten Führungsmagneten (Guidance Magnet) erzeugt. Ein entsprechendes Spulensystem ist z.B. aus der DE 103 40 925 B3 bekannt.

Die vorliegende Erfindung befasst sich mit einer speziellen Anwendung der MGCE, nämlich der Ösophagus-Gaster-Duodenum(deutsch:ÖGD/englisch:EGD)-Endoskopie, bei welcher eben lediglich Speiseröhre und Magen und gegebenenfalls auch der Zwölffingerdarm eines Patienten mit Hilfe der Kapselendoskopie erreichbar sind.

Für eine derartige ÖGD-Untersuchung wird der Magen z.B. zur Hälfte oder mehr mit Wasser gefüllt. Die Kapsel hat ein Volumen von ca. 2-3cm³. Die Endoskopiekapsel mit einer Masse von ca. 2-3g wird mit einer entsprechenden Dichte ausgeführt, welche nahe, bevorzugt knapp unterhalb der von Wasser ist. Die Endoskopiekapsel schwimmt dann im Magen im Wasser und hat dabei zusammen mit ihrem Auftrieb eine effektive Masse von z.B. -0,1g. Die Einbauten in der Kapsel sind so angeordnet, dass sich der Schwerpunkt der Kapsel auf der Kapsellängsachse ein Stück entfernt vom Kapselmittelpunkt befindet. Die länglich geformte Kapsel steht daher im Wasser ohne äußere Krafteinwirkung senkrecht, deren an der Spitze eingebaute Kamera weist im Ruhezustand nach unten.

Durch einen entsprechenden Führungsmagneten kann die Kapsel gegen deren Auftrieb im Magen von der Wasseroberfläche nach unten gezogen werden, horizontal verfahren und gekippt werden. Bei dieser entsprechenden Spezialanwendung der MGCE sind nur wesentlich geringere Kräfte auf die Kapsel auszuüben, als wenn diese durch den gesamten Intestinaltrakt des Patienten bewegt werden soll.

Im Vergleich zum oben genannten allgemeinen Spulensystem wird für die vorliegende ÖGD-Anwendung daher nur ein sogenannter ÖGD-Führungsmagnet (englisch:EGD Guidance Magnet) verwendet, welcher wesentlich einfacher aufgebaut sein kann als der in der DE 103 40 925 B3 beschriebene. Aus der DE 10 2008 004 871 A1 ist z.B. eine Spulenanordnung zur berührungslosen Führung einer Endoskopiekapsel bekannt. Bei einem entsprechenden Magnetsystem wird außerdem beispielsweise auf ein Spulenkühlkonzept aus der DE 10 2007 007 801 A1 zurück gegriffen.

Darüber hinaus ist aus der WO 2006/014011 A1 ein alternativer EGD Guidance Magnet bekannt: Das Magnetsystem besteht aus drei Spulen, die zusammen einen sogenannten Blockmagneten bilden. Dieser Blockmagnet ist unter einem Patiententisch angeordnet und zweidimensional mechanisch unter diesem verfahrbar. Alternativ ist der Patiententisch relativ zum Blockmagneten zweidimensional verfahrbar. Der Zugang zu einem Patienten, welcher auf der Oberseite des Patiententisches angeordnet ist, wird durch den Blockmagneten nicht behindert.

In der US 2007/0270628 A1 ist ein Spulensystem offenbart, bei dem um eine Zentralspule vier Spulen angeordnet sind. Aufgabe der vorliegenden Erfindung ist es, ein weiter verbessertes Spulensystem für eine magnetisch geführte Kapselendoskopie anzugeben.

Die Aufgabe wird gelöst durch ein Spulensystem gemäß Patentanspruch 1, welches zur Erzeugung der Magnetfelder für eine magnetisch geführte Kapselendoskopie dient. Das Spulensystem weist folgende Komponenten auf, wobei sämtliche Komponenten unter, d.h. an der Unterseite eines Patiententisches angeordnet sind, wobei auf der Oberseite ein Patient lagerbar ist. Die Platte des Patiententisches definiert hierbei eine - in der Regel horizontale - Planebene, muss aber selbst nicht plan sein. Die Planebene kann z.B. über die Außenlängskanten des Patiententisches definiert sein.

Das Spulensystem umfasst eine Zentralspule, deren Normalenrichtung senkrecht zur Planebene steht. Mit anderen Worten ist die Zentralspule parallel unterhalb des Patiententisches angeordnet. Das Spulensystem weist außerdem vier ebenfalls unter dem Tisch angeordnete Spulenpaare auf, welche kreuzweise um die zentrale Spule herum angeordnet sind. Jedes Spulenpaar umfasst zwei Einzelspulen, deren Normalenrichtungen parallel zur Planebene verlaufen und zusätzlich zueinander um 90° versetzt orientiert sind. Mit anderen Worten bestehen die Spulenpaare also aus gekreuzten, jeweils senkrecht zur Zentralspule stehenden Einzelspulen. Die Zentralspule und ein Spulenpaar stehen damit paarweise aufeinander senkrecht und decken damit alle Raumrichtungen für magentische Felder ab. Oberhalb und seitlich des Patiententisches befinden sich keine Spulen.

Zur Erläuterung sei ein kartesisches Koordinatensystem (x,y,z) angenommen: Der Patiententisch liegt mit seiner horizontalen Planebene in der (x,z)-Ebene des Koordinatensystems. Die y-Richtung bildet die Vertikale und also die Flächennormale am Patiententisch. Die Zentralspule ist dann auch in y-Richtung orientiert, d.h. die Flächennormale der von der Spule umgespannten Ebene zeigt in y-Richtung. Die Einzelspulen der Spulenpaare sind entsprechend jeweils in x- und z-Richtung orientiert.

Die Erfindung besteht damit in der Idee bzw. Konzeption der Spulengeometrie und -anordnung. Es ergibt sich eine maximale Patientenzugänglichkeit während einer Untersuchung bzw. Prozedur, welche am Patienten auszuführen ist, weil der Magnet, also das gesamte Spulensystem, vollständig unter der Patientenliege angeordnet bzw. "versteckt" ist. Das erfindungsgemäße Spulensystem umfasst nur neun Spulen im Gegensatz zu 10 oder 12 Spulen der bekannten Systeme. Im Gegensatz zum o.g. bekannten Konzept des Blockmagneten ist das Spulensystem starr angeordnet, d.h. der Guidance Magnet enthält keine mechanisch beweglichen Teile.

Gegenüber einem System, welches den Patienten umschließt müssen allerdings Verstärker höherer maximaler Stromstärke und Leistung von ca. 350A bzw. 40kW im Vergleich zu 90A bzw. 10kW für die Spulen eingesetzt werden.

Jede Einzelspule eines Spulenpaares weist einen Abschnitt auf, welcher der Planebene am nächsten gelegen ist, also sich nächstliegend an der Planebene befindet. In einer bevorzugten Ausführungsform der Erfindung verläuft dieser Abschnitt parallel zur Planebene. Die Einzelspule weist somit zumindest einen planen Abschnitt auf, der möglichst nahe am Patiententisch angeordnet ist und somit ein möglichst starkes Feld im Bereich des Patienten erzeugen kann. Die restlichen Spulenteile entfernen sich von der Planebene bzw. dem Patiententisch, und deren Feldanteile treten an der Oberseite des Patiententisches in den Hintergrund.

In einer weiteren bevorzugten Ausführungsform weisen die Zentralspule und/oder die Einzelspulen eine rechteckige Form mit abgerundeten Ecken auf. Die Wicklung der Spulen verläuft also entlang einer Kurve, die annähernd ein Rechteck beschreibt bzw. die von der Wicklung umgrenzte Fläche ist annähernd rechteckig. Insbesondere für die Einzelspulen verfügen diese dann über eine Seiten, also einen Abschnitt, welche nächstliegend der Planebene bzw. Patientenliege angeordnet ist und parallel zu dieser verläuft. An diesen Abschnitt schließen sich dann die beiden senkrecht hierzu verlaufenden Seiten des Rechtecks an, welche jedoch wegen des 90° Winkels im Rechteck um 90° versetzte Feldkomponenten erzeugen. Die vierte Seite des Rechteckes ist dann von der Planebene möglichst weit entfernt, um das vom ersten Abschnitt erzeugte Feld praktisch nur noch geringfügig zu schwächen.

Bei der Ausführung der Zentralspule als Rechteck oder Quadrat können insbesondere die vier Spulenpaare kreuzweise an den Ecken der Zentralspule angeordnet sein. Die Einzelspulen sind dann parallel zu den jeweiligen Seiten der rechteckigen Zentralspule ausgerichtet.

In einer weiteren bevorzugten Ausführungsform der Erfindung ist die jeweils eine Einzelspule eines Spulenpaares in die jeweils andere Einzelspule eingesteckt. Die Herstellung des Spulenpaares ist dadurch besonders einfach möglich, da keine Überkreuzung der Wicklungen nötig ist.

In einer weiteren vorteilhaften Ausführungsform der Erfindung weisen alle Spulenpaare gleich Ausrichtung auf. Mit obiger Nomenklatur einer in y-Richtung orientierten Zentralspule sind dann beispielsweise sämtliche erste Spulen der vier Spulenpaare in x-Richtung und sämtliche zweiten Spulen der vier Spulenpaare in z-Richtung ausgerichtet. So entsteht eine äußert symmetrische Anordnung.

In einer weiteren bevorzugten Ausführungsform ist die Zentralspule und/oder die Einzelspule eine Racetrackspule. Racetrackspulen sind eben und können beispielsweise als Aluminium oder Kupferbandwicklung ausgeführt werden.

In einer bevorzugten Variante weist daher die Racetrackspule eine Bandwicklung auf.

In einer bevorzugten Ausführungsform erfolgt eine Kühlung der Bandwicklung an einer oder an beiden Seitenflächen der Bandwicklung. Bei einseitiger Kühlung erfolgt die Kühlung an derjenigen Seite der Bandwicklung, so dass sich ein möglichst kompaktes Design der Spulenanordnung bei größtmöglicher Nähe zum Arbeitsvolumen ergibt.

Für eine weitere Beschreibung der Erfindung wird auf die Ausführungsbeispiele der Zeichnungen verwiesen. Es zeigen, jeweils in einer schematischen Prinzipskizze:
- Fig. 1: einen Patiententisch mit erfindungsgemäßem Spulensystem in Draufsicht,
- Fig. 2: das Spulensystem in Fig. 1.in Richtung des Pfeils II.

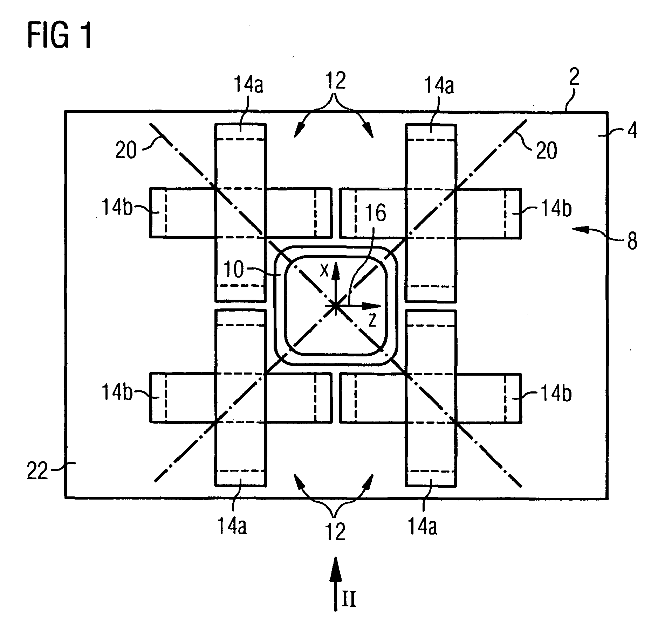

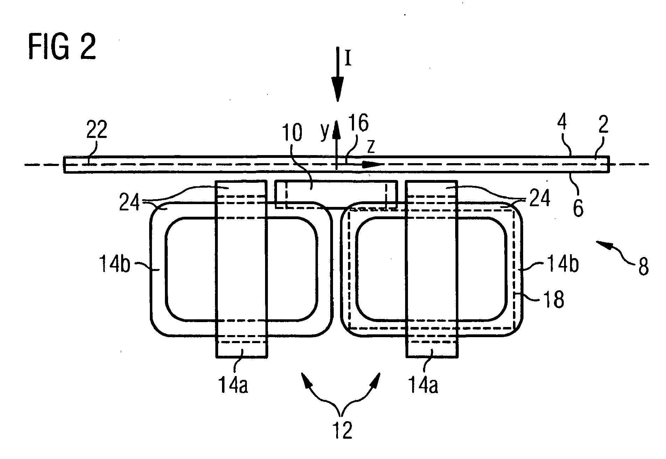

Die Figuren 1 und 2 zeigen jeweils in Richtung der Ansichtspfeile I und II einen Patiententisch 2, auf dessen Oberseite 4 an einem nicht dargestellten Patienten eine magnetisch geführte Kapselendoskopie (MGCE) durchgeführt werden soll. Zur Kraftausübung auf die nicht dargestellte Kapsel ist an der Unterseite 6 des Patiententisches 2 ein Spulensystem 8 ortsfest angebracht. Der Patiententisch bzw. dessen Tischplatte spannt eine Planebene 22 auf.

Das Spulensystem 8 umfasst eine Zentralspule 10 sowie vier Spulenpaare 12, welche jeweils aus einer ersten Einzelspule 14a und einer zweiten Einzelspule 14b bestehen. Die Einzelspulen 14b sind hierbei jeweils in die Einzelspulen 14a eingesteckt. Die Zentralspule 10 ist bezüglich der Orientierung eines Koordinatensystems 16 eine Hy-Spule, d.h. deren Normalenrichtung zeigt in y-Richtung. Die Einzelspulen 14a sind Hz-Spulen, die Einzelspulen 14b Hx-Spulen.

In einer alternativen, nicht dargestellten Ausführungsform sind die Spulen 14a in die Spulen 14b eingesteckt. Im Ausführungsbeispiel weisen sämtliche Einzelspulen 14a,b sowie die Zentralspule 10 jeweils rechteckige Form auf, was insbesondere in Fig. 1 für die Zentralspule 10 und in Fig. 2 für die beiden abgebildeten Einzelspulen 14b zu sehen ist. In Fig. 2 ist die Rechteckform durch die Linie des Rechtecks 18 für die Einzelspule 14b symbolisiert.

Alle Spulen sind als sogenannte Racetrackspulen ausgeführt, d.h. sie sind eben und mit einer Aluminium- oder Kupferbandwicklung ausgeführt. Die im Folgenden angegebenen Spulenabmessungen beziehen sich auf eine Kupferwicklung - welche nicht notwendigerweise eine Bandwicklung sein muss - mit einem angenommenen Füllfaktor einschließlich Kühlung von 85%. Die Außenabmessungen der Zentralspule 10 in x- und z-Richtung beträgt jeweils ca. 60cm. Das Wickelpaket hat eine Breite von ca. 10cm in y-Richtung auf 7,5cm in x- bzw. z-Richtung.

Die Einzelspulen 14a,b haben einen Wicklungsquerschnitt von ca. 25cm auf 9cm, die Außenabmessungen der Einzelspulen 14a sind hierbei ca. 80 bis 100cm in y-Richtung und ca. 80cm in x-Richtung. Die Außenabmessungen der Einzelspulen 14b sind ca. 60 bis 80cm in y-Richtung und ca. 80cm in z-Richtung.

In Fig. 1 ist zu erkennen, wie die Spulenpaare 12 kreuzförmig, angedeutet durch das Kreuz 20, bezüglich der Planebene 22 um die Zentralspule 10 herum gruppiert sind. Das Kreuz 20 trifft hierbei die jeweiligen Ecken der Zentralspule 10. Im Bereich des Patiententisches 2 verlaufen die jeweiligen naheliegendsten, also obersten Abschnitte der Einzelspulen 14a,b jeweils parallel zu einer Seite der Zentralspule 10.

In Fig. 1 und 2 ist auch zu erkennen, das sowohl die Zentralspule 10 parallel zur Planebene 22 liegt als auch die jeweiligen oberen Abschnitte 24 der Einzelspulen 14a,b welche dem Patiententisch am nächsten liegen, jeweils parallel zur Planebene 22 verlaufen.

## Patentansprüche

1. Spulensystem (8) für eine magnetisch geführte Kapselendoskopie, mit einem eine Planebene (22) definierenden Patiententisch (2) und folgenden, unter dem Patiententisch (2) angeordneten Komponenten:
- eine Zentralspule (10) mit Normalenrichtung (y) senkrecht zur Planebene (22),
- vier bezüglich der Planebene (22) nach Art eines Kreuzes (20) um die Zentralspule (10) angeordnete Spulenpaare (12), die jeweils zwei Einzelspulen (14a,b) umfassen, deren Normalenrichtungen (z,x) parallel zur Planebene (22) und zueinander um 90° versetzt orientiert sind.

2. Spulensystem (8) nach Anspruch 1, bei dem die Einzelspule (14a,b) einen der Planebene (22) nächstliegenden Abschnitt (24) aufweist, der parallel zur Planebene (22) verläuft.

3. Spulensystem (8) nach einem der vorhergehenden Ansprüche, bei dem die Zentralspule (10) und/oder die Einzelspule (14a,b) die Form eines Rechtecks (18) aufweist.

4. Spulensystem (8) nach einem der vorhergehenden Ansprüche, bei dem die eine Einzelspule (14b) eines Spulenpaares (12) in die andere Einzelspule (14a) eingesteckt ist.

5. Spulensystem (8) nach einem der vorhergehenden Ansprüche, bei dem alle Spulenpaare (12) gleiche Ausrichtung (z,x) aufweisen.

6. Spulensystem (8) nach einem der vorhergehenden Ansprüche, bei dem die Zentralspule (10) und/oder die Einzelspule (14a,b) eine Racetrackspule ist.

7. Spulensystem (8) nach Anspruch 6, bei dem die Racetrackspule eine Bandwicklung aufweist.

8. Spulensystem (8) nach Anspruch 7, bei dem eine Kühlung der Bandwicklung an einer oder an beiden Seitenflächen der Bandwicklung erfolgt.

## Claims

1. Solenoid system (8) for magnetically guided capsule endoscopy, comprising a patient table (2) defining a flat plane (22) and the following components arranged under the patient table (2):
- a central coil (10) with a normal direction (y) perpendicular to the flat plane (22),
- four coil pairs (12), which, in respect of the flat plane (22), are arranged around the central coil (10) to form a cross (20) and which respectively comprise two single coils (14a, b), the normal directions (z, x) of which are oriented parallel to the flat plane (22) and offset by 90° with respect to one another.

2. Solenoid system (8) according to Claim 1, in which the single coil (14a, b) has a section (24), closest to the flat plane (22), which runs parallel to the flat plane (22).

3. Solenoid system (8) according to one of the preceding claims, in which the central coil (10) and/or the single coil (14a, b) has the shape of a rectangle (18).

4. Solenoid system (8) according to one of the preceding claims, in which the one single coil (14b) of a coil pair (12) is inserted into the other single coil (14a).

5. Solenoid system (8) according to one of the preceding claims, in which all coil pairs (12) have the same alignment (z, x).

6. Solenoid system (8) according to one of the preceding claims, in which the central coil (10) and/or the single coil (14a, b) is a racetrack coil.

7. Solenoid system (8) according to Claim 6, in which the racetrack coil has a strip winding.

8. Solenoid system (8) according to Claim 7, in which the strip winding is cooled on one or on both lateral sides of the strip winding.

## Revendications

1. Système ( 8 ) de bobines pour une endoscopie par capsule à guidage magnétique, comprenant une couchette ( 2 ) de patient définissant un plan ( 22 ) et des éléments suivants, disposés sous la couchette ( 2 ) de patient :
- une bobine ( 10 ) centrale ayant une direction ( y ) normale perpendiculaire au plan ( 22 ),
- quatre paires ( 12 ) de bobines disposées autour de la bobine ( 10 ) centrale, en étant par rapport au plan ( 22 ), à la manière d'une croix ( 20 ), paires qui comprennent respectivement deux bobines ( 14a, b ) individuelles, dont les directions ( z, x ) normales sont parallèles au plan ( 22 ) et sont décalées de 90°, l'une par rapport à l'autre.

2. Système ( 8 ) de bobines suivant la revendication 1, dans lequel les bobines ( 14a, b ) individuelles ont une partie ( 24 ) la plus proche du plan ( 22 ) qui s'étend parallèlement au plan ( 22 ).

3. Système ( 8 ) de bobines suivant l'une des revendications précédentes, dans lequel la bobine ( 10 ) centrale et/ou la bobine ( 14a, b ) individuelle a la forme d'un rectangle ( 18 ).

4. Système ( 8 ) de bobines suivant l'une des revendications précédentes, dans lequel l'une des bobines ( 14b ) individuelles d'une paire ( 12 ) de bobines est emmanchée dans l'autre bobine ( 14a ) individuelle.

5. Système ( 8 ) de bobines suivant l'une des revendications précédentes, dans lequel toutes les paires ( 12 ) de bobines ont la même orientation ( z, x ).

6. Système ( 8 ) de bobines suivant l'une des revendications précédentes, dans lequel la bobine ( 10 ) centrale et/ou la bobine ( 14a, b ) individuelle est une bobine racetrack.

7. Système ( 8 ) de bobines suivant la revendication 6, dans lequel la bobine racetrack a un enroulement en ruban.

8. Système ( 8 ) de bobines suivant la revendication 7, dans lequel un refroidissement de l'enroulement en ruban s'effectue sur l'une ou sur les deux surfaces latérales de l'enroulement en ruban.
